# FASCICULE DE BREVET EUROPEEN

(11) **EP 2 185 148 B1**
(45) Date de publication et mention de la délivrance du brevet: **12.06.2013**
(21) Numéro de dépôt: 08829362.6
(22) Date de dépôt: 28.08.2008
(51) Int. Cl.: A61K 31/23, A61P 17/00, A61K 8/37, A61Q 7/02, A61K 31/5575

(54) **UTILISATION DE TRAVOPROST POUR TRAITER LA CHUTE DES CHEVEUX**
VERWENDUNG VON TRAVOPROST ZUR BEHANDLUNG VON HAARVERLUST
USE OF TRAVOPROST FOR TREATING HAIR LOSS

(30) Priorité: 28.08.2007 FR 0757208
(43) Date de publication de la demande: 19.05.2010
(60) Demande divisionnaire: 12191293.5
(73) Titulaire: Galderma Research & Development, 06410 Biot (FR)
(72) Inventeur: RETHORE, Sandrine, F-06560 Valbonne (FR); ROSIGNOLI, Carine, F-06410 Biot (FR); JOMARD, André, F-06460 Saint Vallier de Thiey (FR); VOEGEL, Johannes, F-06740 Chateauneuf/Grasse (FR)
(74) Mandataire: Starck-Loudes, Anne-Caroline
(86) Numéro de dépôt international: PCT/FR2008/051539
(87) Numéro de publication internationale: WO 2009/030862

(56) Documents cités:
- EP-A- 1 739 083
- EP-A- 1 775 294
- WO-A-03/009820
- US-A1- 2007 160 562
- BARNEBEY ET AL: "The Safety and Efficacy of Travoprost 0.004%/Timolol 0.5% Fixed Combination Ophthalmic Solution" AMERICAN JOURNAL OF OPHTHALMOLOGY, OPHTHALMIC PUBL., CHICAGO, IL,, US, vol. 140, no. 1, juillet 2005 (2005-07), pages 1e1-1e8, XP005114329 ISSN: 0002-9394
- FELETTI F ET AL: "Periocular pigmentation associated with use of travoprost for the treatment of alopecia areata of the eyelashes [22]" JEADV. JOURNAL OF THE EUROPEAN ACADEMY OF DERMATOLOGY AND VENEREOLOGY, ELSEVIER SCIENCE PUBLISHERS, AMSTERDAM, NL, vol. 21, no. 3, 2007, pages 421-423, XP008090297 ISSN: 0926-9959

## Description

La présente invention concerne la préparation de compositions destinées à induire ou stimuler la croissance des tiges pilaires et en particulier des cheveux et des cils humains.

Chez l'être humain, la croissance des cheveux est cyclique et comporte trois phases successives : la phase anagène, la phase catagène et la phase télogène. Chaque follicule de la chevelure se renouvelle donc en permanence, de façon cyclique et indépendante aux follicules adjacents. La phase anagène ou phase de croissance, au cours de laquelle les cheveux s'allongent, dure plusieurs années. La phase catagène qui succède à la phase anagène est très courte et ne dure que quelques semaines. Au cours de cette phase, le cheveu subit une involution, le follicule s'atrophie et son implantation dermique apparaît de plus en plus haute. La phase télogène, qui dure quelques mois, correspond à une période de repos du follicule où le cheveu finit par tomber. Après cette phase de repos un nouveau follicule est régénéré, sur place, et un nouveau cycle recommence.
A chaque instant, tous les cheveux ne sont pas dans la même phase au même moment. Ainsi, sur les 150 000 cheveux environ que comporte une chevelure, seuls 10% d'entre eux environ sont au repos et seront donc remplacés en quelques mois selon une horloge biologique propre à chaque cheveu.
Chez la souris et les autres mammifères à fourrure, les follicules pileux possèdent également un cycle de renouvellement comportant les trois phases anagène, catagène et télogène, toutefois les cycles pilaires sont souvent « synchronisés » c'est-à-dire dans la même phase du cycle au même moment au sein d'une même région.

La chute ou perte naturelle des cheveux peut être estimée, en moyenne, à quelques cent cheveux par jour pour un état physiologique normal. Cependant, il arrive que le cycle pilaire puisse se dérégler et que la chute des cheveux s'accélère et conduise à une perte temporaire ou définitive des cheveux appelée alopécie. Différentes causes peuvent être à l'origine d'une alopécie.
Il peut s'agir d'une perte massive ou d'altérations des cheveux appelées telogen effluvium, au décours d'une grossesse, au cours d'états de dénutrition ou de déséquilibres alimentaires, ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers. Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

Il peut également s'agir d'une alopécie qui est essentiellement due à une perturbation du renouvellement capillaire qui entraîne, dans un premier temps, l'accélération de la fréquence des cycles aux dépens de la qualité des cheveux puis de leur quantité. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme, et chez les femmes, on constate une alopécie diffuse du vertex.
Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.
Il est connu, par ailleurs, que certains facteurs tels qu'un déséquilibre hormonal, un stress physiologique, la malnutrition, peuvent accentuer le phénomène.
Le terme alopécie recouvre aussi toute une famille d'atteintes du follicule pileux ayant pour conséquence finale la perte définitive partielle ou générale des cheveux.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute.
Dans cette optique, on a déjà proposé un grand nombre de compositions comprenant des actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.
Des études cliniques ont démontré que des analogues de PGF2 alpha avaient la propriété de provoquer la croissance de poils et de cils chez l'homme et chez l'animal (Murray A and Johnstone MD, 1997. Am J Opht, 124(4), 544-547). Chez l'homme, des essais réalisés sur le cuir chevelu ont montré qu'un analogue de prostaglandine E2 (le viprostol) avait la propriété d'augmenter la densité capillaire (Roenigk HH., 1988. Clinic Dermatol, 6(4), 119-121).
Le brevet WO 98/33497 décrit des compositions pharmaceutiques contenant des prostaglandines ou des dérivés de prostaglandines destinées à lutter contre la chute des cheveux chez l'homme. Les prostaglandines du type A2, F2 alpha et E2 sont préférées.
Le Travoprost, seul ou en combinaison, a été proposé pour stimuler la pousse des cheveux en administration quotidienne (WO03/009820, EP1352629, EP1358868 US 2007160562).
Toutefois, il ressort de ce qui précède que la majorité des produits destinés à lutter contre la chute des cheveux et/ou à induire la repousse des cheveux présentent une posologie astreignante pour le patient car l'administration doit être réalisée de manière quotidienne ou bi-quotidienne. En particulier, la posologie recommandée pour le Minoxidil à la dose de 2% est une administration bi-quotidienne.
Ainsi, un produit d'efficacité comparable ou supérieure à celle des produits déjà existants mais administré moins fréquemment soit de manière non quotidienne à hebdomadaire voire bimensuelle présenterait un avantage important pour le patient en termes de confort et de sécurité d'utilisation.
Il existe donc un réel besoin d'identifier un produit avec une posologie moins contraignante qui ne nécessiterait pas une administration quotidienne ou bi-quotidienne pour être aussi, voire plus efficace que les produits de référence.
La Demanderesse s'est maintenant aperçue que le Travoprost administré de façon non quotidienne permettait d'induire une croissance des tiges pilaires comparable voire supérieure à celle obtenue avec une administration quotidienne. En effet, dans les exemples présentés, la Demanderesse a mis en évidence de façon surprenante que le Travoprost administré en traitement unique induisait une croissance des follicules pileux humains *in vitro* comparable à celle observée après un traitement répété par le composé pendant 7 jours (voir figures 1 et 3). D'autre part, la Demanderesse a également mis en évidence in vivo, de façon surprenante, qu'une administration unique de Travoprost était plus efficace sur la repousse des tiges pilaires que des administrations quotidiennes (voir figures 4 et 5).

L'invention a donc pour objet l'utilisation de Travoprost dans ou pour la fabrication d'une composition ; le Travoprost ou la composition étant destinée à stimuler ou induire la croissance et/ou à diminuer la chute et/ou augmenter la densité et/ou réduire l'hétérogénéité du diamètre des tiges pilaires chez l'être humain, ladite composition étant destinée à être appliquée selon un intervalle de temps entre deux applications supérieur à 24 heures, tel que revendiqué, et comprenant une quantité de travoprost comprise entre 0,005 et 0,01%.
Plus précisément, l'invention a pour objet l'utilisation du Travoprost pour la préparation d'un médicament à application topique, pour stimuler ou induire la croissance et/ou diminuer la chute et/ou augmenter la densité et/ou réduire l'hétérogénéité du diamètre des tiges pilaires chez l'être humain, caractérisé en ce que le régime d'administration du médicament comprend un intervalle de temps entre deux applications supérieur à 24 heures, tel que revendiqué et comprenant une quantité de travoprost comprise entr 0,005 et 0,01%.
Dans le cadre de la présente invention les termes posologie (de l'application de la composition) et régime d'administration sont équivalents.
L'application pouvant être effectuée de manière non quotidienne c'est-à-dire tous les deux, trois, quatre, cinq, six ou sept jours. De même la présente invention vise également l'application hebdomadaire, bi-hebdomadaire, tri-hebdomadaire, mensuelle, bi-mensuelle ou tri-mensuelle.
Les tiges pilaires sont préférentiellement choisies parmi les cheveux, les poils et les cils. La composition est un médicament.
Selon un premier mode de réalisation, la composition, plus particulièrement un médicament, est destinée à être appliquée avec un intervalle de temps entre deux applications supérieur à 24 heures tel que revendiqué. Dans ce cas l'application topique n'est plus effectuée tous les jours (administrations quotidiennes ou bi-quotidiennes), mais au contraire elle est effectuée tous les deux, trois, quatre, cinq, six ou sept jours et doit donc être considérée comme faisant partie du champ de la présente invention.
Selon un autre mode de réalisation, la composition, plus particulièrement un médicament, est destinée à être appliquée en une application hebdomadaire.
Selon un autre mode de réalisation de l'invention, la composition, plus particulièrement un médicament, est destinée à être appliquée deux (bi-hebdomadaire) ou trois fois (tri-hebdomadaire) par semaine.
Selon un autre mode de réalisation, la composition, plus particulièrement un médicament, est destinée à être appliquée en une application une fois par mois (mensuel).
Selon un autre mode de réalisation, la composition, plus particulièrement un médicament, est destinée à être appliquée une, deux fois (ou bi-mensuel) ou trois fois par mois.
Dans le cadre de la présente invention, il peut être envisagé d'administrer la composition, plus particulièrement un médicament, plusieurs fois selon l'échelle de temps défini (hebdomadaire ou mensuelle). Le praticien adaptera donc la durée du traitement en fonction du résultat recherché, le traitement pouvant ainsi s'étaler sur plusieurs mois.

La composition, plus particulièrement un médicament, comprend une quantité de travoprost , comprise entre 0,005 et 0,01%.

La présente invention se rapporte donc à l'utilisation selon Travoprost pour la préparation d'un médicament destiné à être appliquer de manière topique.
Selon un mode de réalisation préféré, la composition, plus particulièrement un médicament, comprend environ 0.01% de Travoprost et est destinée à être appliquée en une application topique.

La composition, plus particulièrement un médicament, est de préférence sous la forme d'une lotion, d'une crème, d'une pommade, d'une poudre ou d'un shampoing ou après-shampoing.
Selon l'un de ses modes de réalisation, les compositions sont destinées à prévenir, diminuer ou ralentir la chute des cheveux, notamment au traitement de l'alopécie, en particulier de l'alopécie androgénétique.
L'utilisation cosmétique de Travoprost dans une composition cosmétique de soin capillaire pour l'être humain ou pour la préparation d'une composition capillaire pour l'être humain destinée à traiter l'alopécie androgénique est ici divulguée. Ainsi, cette composition permet de maintenir en bon état la chevelure et/ou lutter contre la chute naturelle des cheveux chez l'Homme. Ainsi, un procédé cosmétique pour lutter contre la chute naturelle des cheveux et/ou maintenir la chevelure en bon état, comprenant l'application topique du Travoprost, l'intervalle de temps entre 2 applications étant supérieur à 24 heures est ici divulguée.
Un autre objet de l'invention est une composition pour le traitement de l'alopécie androgénique, comprenant l'application d'une composition de soin capillaire comprenant du Travoprost, à un Homme nécessitant un tel traitement, ladite composition étant destinée à être appliquée avec un intervalle de temps entre deux applications supérieur à 24 heures, tel que revendiqué.
Dans le cadre du traitement de l'alopécie androgénique, la composition est destinée à être appliquée tous les deux, trois, quatre, cinq, six ou sept jours.
Selon un mode alternatif de l'invention, la composition est destinée à être appliquée en une application hebdomadaire, bi- hebdomadaire, tri-hebdomadaire, mensuelle, bi- ou tri- mensuelle. Ladite composition comprend une quantité de Travoprost; comprise entre 0,005 et 0,01%.

La présente invention vise donc l'application non quotidienne du Travoprost ce qui supprime une grande contrainte. La fréquence d'administration est plus réduite et ainsi la posologie est différente de ce qui est habituellement décrit. Ainsi l'application topique de la composition selon l'invention peut être effectuée tous les deux, trois, quatre, cinq, six ou sept jours. De même la présente invention vise également l'application hebdomadaire, bi-hebdomadaire, tri-hebdomadaire, mensuelle, bi-mensuelle ou tri-mensuelle.

Il peut également être envisagé un traitement séquentiel dans lequel la fréquence sera définie par séquence de traitement. On peut dans ce cas envisager une première séquence de traitement avec une fréquence particulière (par exemple hebdomadaire) suivie d'une seconde séquence de traitement avec une fréquence différente (par exemple bi-mensuelle) comme par exemple un traitement défini par un praticien à raison de 3 mois avec une application 1 fois par semaine, puis 3 mois avec une application une fois par mois. Différentes séquences sont possibles ; elles seront définies par le praticien au cas par cas.

Le Travoprost est un analogue de prostaglandine, qui répond à la formule suivante :

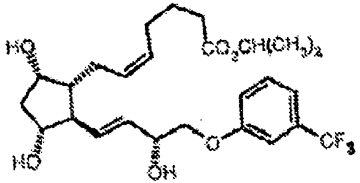

Il s'agit du 13, 14-dihydrofluprostenol isopropyl ester connu également sous le nom chimique propan-2-yl (E)-7-[(1R,2S,3R,5S)-3,5-dihydroxy-2-[(E)-3-hydroxy-4-[3-( trifluoromethyl)phenoxy]but-1-enyl]cyclopentyl]hept-5-enoate, connu également sous le nom chimique isopropyl (Z)-7- [(1 R ,2 R ,3 R ,5 S)-3,5-dihydroxy-2-[(1 E ,3 R)-3-hydroxy-4-[(α,α,α-trifluoro- m -tolyl)oxy]-1- butenyl]cyclopentyl]-5-heptenoate. Les acides correspondants du Travoprost sont également utilisables selon l'invention.

Par « hétérogénéité des diamètres des cheveux » on entend une grande variation des diamètres des cheveux sur une même zone du scalp ; certains cheveux ayant un diamètre physiologique de 0,05 à 0,1 mm, d'autres ayant, au voisinage immédiat de ces cheveux, un diamètre diminué (cheveux fins). Ainsi par "réduire l'hétérogénéité des diamètres", on entend augmenter le diamètre des cheveux fins.

Par « augmenter la densité pilaire » on entend augmenter le nombre de tiges pilaires, cheveux ou cils par cm² de peau ou de cuir chevelu.

Par « application topique », on entend une application sur les phanères, la peau, notamment le cuir chevelu, les poils-et les cheveux.

Avantageusement, une composition selon l'invention comprendra des excipients adaptés à une administration sur la zone cutanée ou la tige pilaire visée, et notamment le cuir chevelu, les cheveux, les poils ou les cils. Le milieu dans lequel se trouvera le Travoprost selon l'invention peut être anhydre ou aqueux. La composition comprend par exemple un milieu physiologiquement acceptable pouvant être constitué d'eau ou d'au moins un solvant choisi parmi les solvants organiques hydrophiles, les solvants organiques lipophiles, les solvants organiques amphiphiles et leurs mélanges, notamment un mélange d'eau et d'au moins l'un des solvants précités.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H) ou multiples, d'une poudre libre ou compactée à utiliser telle quelle ou à incorporer dans un milieu physiologiquement acceptable, ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elle peut être anhydre ou aqueuse. Elle peut également consister en des préparations solides constituant des savons ou des pains de nettoyage. Ces compositions sont préparées selon les méthodes usuelles.

La composition utilisable selon l'invention peut en particulier consister en une composition pour soins capillaires, et notamment un shampooing ou un après shampoing en particulier d'application bi- ou tri-hebdomadaire ou hebdomadaire, une lotion de mise en plis, une lotion traitante, une lotion de soin capillaire par exemple d'application tous les deux jours ou bi-hebdomadaire, une crème ou un gel coiffant, des lotions restructurantes pour les cheveux, un masque, etc.

La composition cosmétique utile dans l'invention est préférentiellement une crème, une lotion capillaire, un shampoing ou un après-shampoing, un mascara capillaire ou pour cils.

Pour une application sur les cils ou les poils, la composition selon l'invention peut se présenter sous forme d'un mascara, pigmenté ou non, à appliquer à la brosse ou au peigne sur les cils, les sourcils, les cheveux, ou encore sur les poils de barbe ou de moustache.

Les quantités des différents constituants des compositions utilisables selon l'invention sont celles classiquement utilisées dans les domaines considérés.
La phase aqueuse contient de l'eau et éventuellement un ingrédient miscible en toute proportion à l'eau comme les alcools inférieurs en C1 à C8 tel que l'éthanol, l'isopropanol, les polyols comme le propylène glycol, le glycérol, le sorbitol ou encore l'acétone ou l'éther.
Lorsque la composition utilisable selon l'invention est une émulsion, la proportion de la phase grasse peut aller de 2 à 80%, notamment de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,1% à 30% en poids, notamment de 0,3% à 30% en poids, de préférence de 0,5 à 20% en poids, et encore mieux de 1 à 8% par rapport au poids total de la composition. L'émulsion peut, en outre, contenir des vésicules lipidiques et notamment des liposomes.
Lorsque la composition utilisable selon l'invention est une solution ou un gel huileux, la phase grasse peut représenter plus de 90% du poids total de la composition.

Avantageusement, la composition comprend des microsphères, des nanosphères, des liposomes, des oléosomes ou des nanocapsules, dans lesquels le Travoprost est encapsulé. Des exemples de telles formulations sont décrits notamment dans les brevets, EP 375520, EP447318, EP557489, WO 97/12602, EP1151741 ou US 5 914126.

A titre d'exemple, les microsphères pourront être préparées selon la méthode décrite dans la demande de brevet EP 0 375 520.

Les nanosphères peuvent se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

Le Travoprost peut aussi être encapsulé dans des nanocapsules consistant en un enrobage lamellaire obtenu à partir d'un tensio-actif siliconé tel que décrit dans la demande de brevet EP 0 780 115; les nanocapsules pourront également être préparées à base de polyesters sulfonique hydrodispersibles selon par exemple la technique décrite dans la demande de brevet FR 0113337.

Avantageusement pour une application capillaire, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est une dispersion de cire-dans-eau ou de cire dans huile, une huile gélifiée, un gel aqueux, pigmenté ou non.

La composition utile dans l'invention peut contenir également des adjuvants habituels dans le domaine cosmétique, tels que les gélifiants ou épaississants hydrophiles ou lipophiles, les additifs hydrophiles ou lipophiles, les conservateurs, les antioxydants, les solvants, les parfums, les charges, les filtres, les absorbeurs d'odeur les électrolytes, les neutralisants, les agents bloqueurs d'U.V. comme les filtres solaires, les polymères filmogènes, les actifs cosmétiques et pharmaceutiques à action bénéfique pour la peau ou les tiges pilaires et les matières colorantes, solubles ou non dans le milieu. Les quantités de ces différents adjuvants sont celles classiquement utilisées dans le domaine cosmétique, et notamment de 0,01 à 50% du poids total de la composition, par exemple de 0,01% à 20%, notamment inférieure ou égale à 10% du poids total de la composition, et en particulier supérieure ou égale à 0,1%. Ces adjuvants, selon leur nature, peuvent être introduits dans la phase grasse, dans la phase aqueuse et/ou dans les sphérules, vésicules ou microsphères lipidiques, telles que les liposomes.
La phase grasse peut contenir des composés gras ou huileux, liquides à température ambiante (25 DEG C) et pression atmosphérique (760 mm de Hg), généralement appelés huiles. Ces huiles peuvent être compatibles ou non entre elles et former une phase grasse liquide macroscopiquement homogène ou un système bi- ou triphasique.
La phase grasse peut, en plus des huiles, contenir des cires, des gommes, des polymères lipophiles, des produits "pâteux" ou visqueux contenant des parties solides et des parties liquides.
Comme huiles ou cires utilisables dans l'invention, on peut citer les huiles minérales (huile de vaseline, isoparaffine hydrogénée), les huiles végétales (fraction liquide du beurre de karité, huile de tournesol, de soja, de blé), les huiles animales (perhydrosqualène), les huiles de synthèse (huile de Purcellin, esters d'acides gras), les huiles ou cires siliconées (polydiméthylsiloxanes linéaires ou cycliques, cyclométhicone, phényltriméthicone) et les huiles fluorées (perfluoropolyéthers), les cires d'abeille, de candelila, carnauba ou paraffine. On peut ajouter à ces huiles et cires des alcools gras et des acides gras libres (acide stéarique, linoléique, linolénique).
Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges; le stéarate ou oléate de sorbitol polyoxyéthyléné, par exemple le polysorbate 60 et le mélange de PEG-6/PEG-32/Glycol Stéarate vendu sous la dénomination de Tefose TM 63 par la société Gattefosse, le monostéarate ou monolaurate de polyéthylène glycol, les diméthiconecopolyols et leurs mélanges.
Comme solvants utilisables dans l'invention, on peut citer les alcools inférieurs, notamment l'éthanol et l'isopropanol, le propylène glycol.
Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones TM , les sels métalliques d'acides gras comme les stéarates d'aluminium et la silice hydrophobe, éthylcellulose, polyéthylène.

Dans la suite du texte, et sauf indication contraire, les quantités des différents ingrédients de la composition sont données en pourcentage en poids par rapport au poids total de la composition.
Une composition préférée utile dans l'invention est une composition à appliquer sur le cuir chevelu.
Selon un mode de réalisation avantageux, ces compositions comprennent en outre au moins un agent bénéfique pour les cheveux tels que notamment les silicones, les huiles végétales, animales, minérales ou de synthèse, les cires, les céramides, les pseudocéramides, les polymères cationiques, les filtres solaires, les vitamines.
Les silicones utilisables conformément à l'invention sont en particulier des polyorganosiloxanes insolubles dans la composition et peuvent se présenter sous forme d'huiles, de cires, de résines ou de gommes.
Les organopolysiloxanes sont définis plus en détail dans l'ouvrage de Walter NOLL "Chemistry and Technology of Silicones" (1968) Academie Press. Elles peuvent être volatiles ou non volatiles.
Le Travoprost est de préférence le seul principe actif pour favoriser la repousse et/ou limiter la chute des cheveux. Cependant il peut être aussi associé à d'autres composés actifs pour favoriser la repousse et/ou limiter la chute des cheveux.
Ces composés peuvent notamment être choisis parmi les inhibiteurs de lipoxygénase tels que décrits dans EP648488, les inhibiteurs de bradykinine décrits notamment dans EP 845700, les prostaglandines et leurs dérivés notamment ceux décrits dans WO 98/33497, WO 95/11003, JP 97-100091, JP 96134242, les agonistes ou antagonistes des récepteurs des prostaglandines, les analogues non prostanoïques de prostaglandines tels que décrits dans EP 1175891 et EP1175890, WO 01/74307, WO 01/74313, WO 01/74314, WO 01/74315 ou WO 01/72268.
Des agents favorisant la pousse du cheveu pouvant être présents dans les compositions selon l'invention incluent les vasodilatateurs, les anti-androgènes, les cyclosporines et leurs analogues, les anti-microbiens, les antifongiques, les anti-inflammatoires à l'exception des inhibiteurs sélectif de la prostaglandine H synthase 1 ou COX-1, les tri-terpènes, seuls ou en mélange.
Les vasodilatateurs tels que les agonistes des canaux potassium incluant le Minoxidil et ses dérivés, l'aminexil et ses dérivés tels que les composés décrits dans les brevets US 3 382247, 5 756092, 5 772990, 5 760043, 5 466694, 5 438058, 4 973474, la chromakalim et le diaxozide ou le nicorandil peuvent ainsi être présents dans les compositions.
Les anti-androgènes utilisables incluent notamment les inhibiteurs de 5 alpha réductase, comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226, le RU58841 et le Casodex.
Les composés anti-microbiens peuvent être choisis parmi les dérivés du sélénium, le kétoconazole, l'octopirox, le triclocarban, le triclosan, le pyrithione zinc, l'itraconazole, l'acide asiatique, l'hinokitiol, mipirocine, et les composés décrits dans EP680745, le chlorhydrate de clinycine, le peroxyde de benzoyle ou de benzyl et la minocycline. Les anti-inflammatoires pourront être choisis parmi les inhibiteurs spécifiques de la Cox-2 comme par exemple le NS-398 et le DuP-697 (B.Batistini et al., DN&P 1994 ; 7(8) :501-511) et/ou les inhibiteurs des lipoxygénases, notamment de 5-lipoxygénase comme par exemple le zileuton (F.J.Alvarez & R.T.Slade, Pharmaceutical Res. 1992 ; 9(11) :1465-1473).
D'autres composés actifs pour favoriser la pousse et/ou limiter la chute des cheveux présents dans les compositions pourront également être choisi dans le groupe comprenant le 6-O-[(9Z, 12Z)-octadéc-9,12-diénoyl]hexapyranose tels que décrits dans FR027293, FR0212828, chlorure de benzlakonium, chlorure de benzethonium, phénol, oestradiol et notamment le 17-α oestradiol ou le 17-β oestradiol, maléate de chlorphéniramine, les dérivés de chlorophylline, cholestérol, cystéine, méthionine, nicotinate de benzyle, menthol, huile de menthe poivrée, panthoténate de calcium, panthénol, résorcinol, les inhibiteurs de la protéine kinase C, les activateurs de la prostaglandine H synthase 1 ou COX-1 tels que décrits dans FR2732597 les inhibiteurs de la glycosidase, les inhibiteurs de glycosaminoglycanase, les esters d'acide pyroglutamique, les acides hexosaccharidiques ou acyl-hexosaccharique, éthylènes aryl substitués, les amino acides N-acylés, les flavonoïdes, les dérivés et analogues d'ascomycine, les antagonistes d'histamine, les triterpènes comme l'acide ursolique et les composés décrits dans US 5529769, US 5468888, US 5631282, les saponines, les inhibiteurs de protéoglycanase, les agonistes et antagonistes d'estrogènes, pseudotèrines, les cytokines et les promoteurs de facteurs de croissance, les inhibiteurs d'IL-1 ou d'IL-6, les promoteurs d'IL-10, les inhibiteurs de TNF, les vitamines, comme la vitamine D, les analogues de la vitamine B12 et le panthoténol, les hydroxyacides, benzophénones, les acides gras estérifiés tels que décrits dans FR027293, FR0212828 et l'hydantoïne.
Selon un mode de réalisation avantageux de l'invention, le Travoprost est associé à un autre composé actif pour favoriser la pousse et/ou limiter la chute des cheveux, tel qu'un composé choisi parmi les prostaglandines, notamment la prostaglandine PGE1, PGE2, leurs sels, leurs esters, leurs analogues et leurs dérivés, notamment ceux décrits dans WO 98/33497, WO 95/11003, JP 97-100091, JP 96134242, en particulier les agonistes des récepteurs des prostaglandines. Elle peut notamment contenir au moins un composé tel les agonistes (sous forme acide ou sous forme d'un précurseur notamment sous forme ester) du récepteur de la prostaglandine F2 alpha (FP-R) à l'exemple du latanoprost, du fluprostenol, du cloprostenol, le bimatoprost, l'unoprostone, les agonistes (et leurs précurseurs notamment les esters) des récepteurs de la prostaglandine E2 (EP1-R, EP2-R, EP3-R, EP4-R) tel le 17 phényle PGE2, le viprostol, le butaprost, le misoprostol, le sulprostone, le 16,16 diméthyle PGE2, 11 désoxy-PGE1, le 1 désoxy PGE1, les agonistes et leurs précurseurs notamment les esters du récepteur de la prostacycline (IP) tel le cicaprost, l'iloprost, l'isocarbacycline, le beraprost, les agonistes et leurs précurseurs notamment les esters du récepteur de la prostaglandine D2 tel le BW245C ((4S)-(3-[(3R,S)-3-cyclohexyl-3-isopropyl]-2,5-dioxo)4-imidazolidine-heptanoic acid), le BW246C ((4R)-(3-[(3R,S)-3-cyclohexyl-3-isopropyl]-2,5-dioxo)4-imidazolidineheptanoic acid), les agonistes et leurs précurseurs notamment les esters du récepteur aux thromboxanes A2 (TP) tel le I-BOP ([1S-[1a,2a(Z), 3b(1E,3S),4a]]-7-[3-[3-hydroxy-4-[4-(iodophenoxy)-1-butenyl]-7-oxabicyclo[2.2.1]hept-2-yl]-5-heptenoic acid).

Selon un autre mode de réalisation, la composition utile selon l'invention comprend, en plus du Travoprost, avantageusement au moins un inhibiteur de la 15-PGDH et/ou au moins une prostaglandine ou un dérivé de prostaglandine comme par exemple les prostaglandines de la série 2 dont notamment PGF 2 alpha et PGE 2 sous forme salines ou sous forme de précurseurs, notamment des esters (exemple les isopropyl esters), leurs dérivés comme le 16,16 dimethyl PGE2, le 17 phényl PGE2, le 16,16 diméthyle PGF2 alpha , le 17 phényl PGF2 alpha les prostaglandines de la série 1 comme le 11 déoxy prostaglandine E1, le 1 déoxy prostaglandine E1 sous forme salines ou esters, leurs analogues notamment le latanoprost, le fluprostenol, l'unoprostone, le bimatoprost, le cloprosténol, le viprostol, le butaprost, le misoprostol, leurs sels ou leurs esters.

De manière préférée, la composition contient au moins un agoniste du récepteur FP tel que décrit par exemple dans WO03/009820 ou un agoniste prostanoïque ou non prostanoïque des récepteurs EP2 et/ou EP4 notamment tel que décrit dans EP 1175892.

Les compositions utiles dans l'invention peuvent également contenir des agents accélérateurs de pénétration. Ces composés sont connus de l'homme du métier et améliorent le passage des agents vers le site d'action; ils seront classiquement présents dans les compositions à des concentrations supérieures ou égales à 0,01 %, notamment de 0,1 à 20% et de préférence de 0,1 à 5%. en poids par rapport au poids total de la composition. Des composés de ce type utilisable sont par exemple l'urée et les composés cités dans la demande WO01/74313.

Les exemples qui suivent sont destinés à illustrer l'invention.

### Légendes des figures :

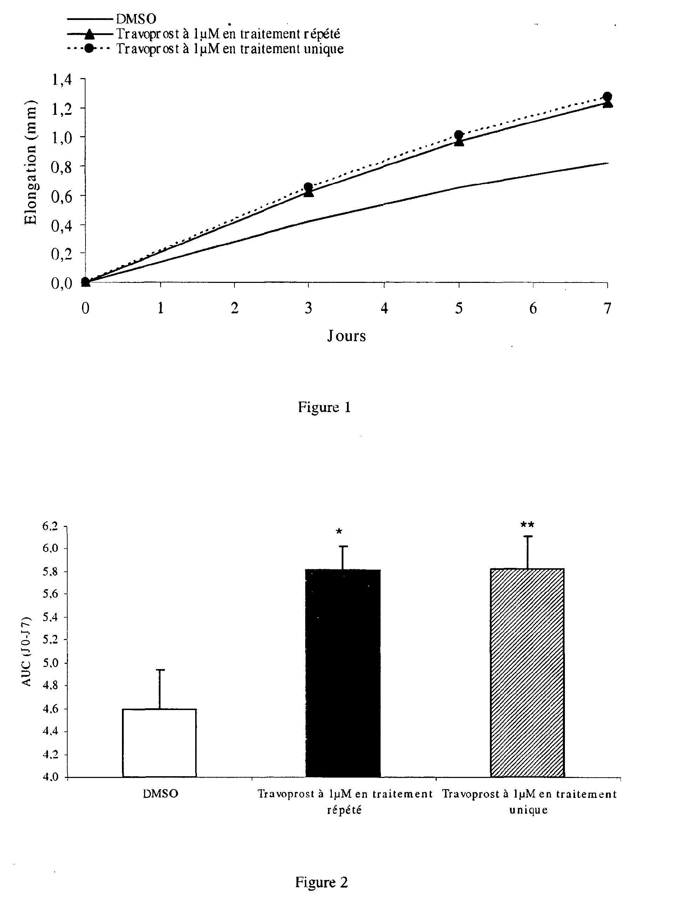

La figure 1 est un graphique comparant l'effet du Travoprost acide à 1µM après traitement unique au moment de la mise en culture et après traitement répété durant 7 jours sur l'élongation de la tige pilaire de follicules pileux humains en survie (exprimée en mm) en fonction du temps.
La figure 2 est un graphique comparant l'effet du Travoprost acide à 1µM après traitement unique au moment de la mise en culture et après traitement durant 7 jours sur l'élongation de la tige pilaire de follicules pileux humains en survie représentée par l'aire sous la courbe de J0 à J7.

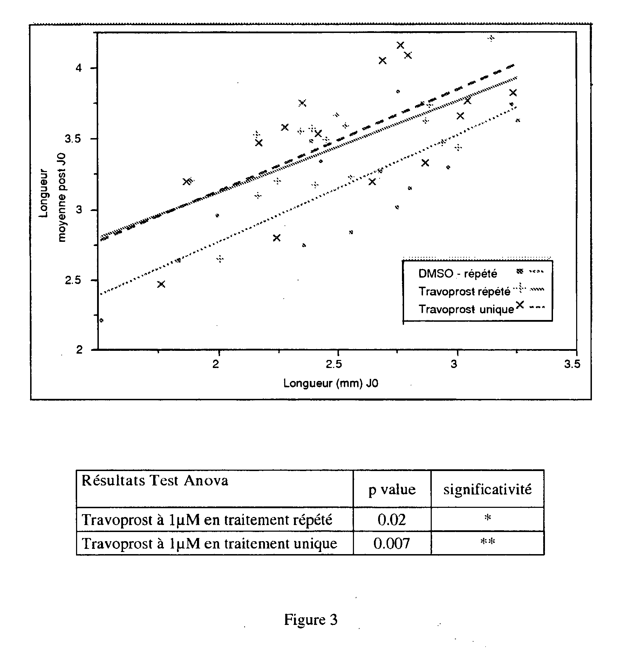

La figure 3 est un graphique montrant l'analyse statistique de l'expérience décrite dans la figure 1. L'analyse statistique est réalisée en comparant l'effet du Travoprost acide à 1µM dans le DMSO après traitement unique et répété par rapport au groupe véhicule traité par le DMSO à 0.1%. p>0.05=NS; 0.01<p<0.05=*;0.001<p < 0.01 = ** ; p < 0.001=***

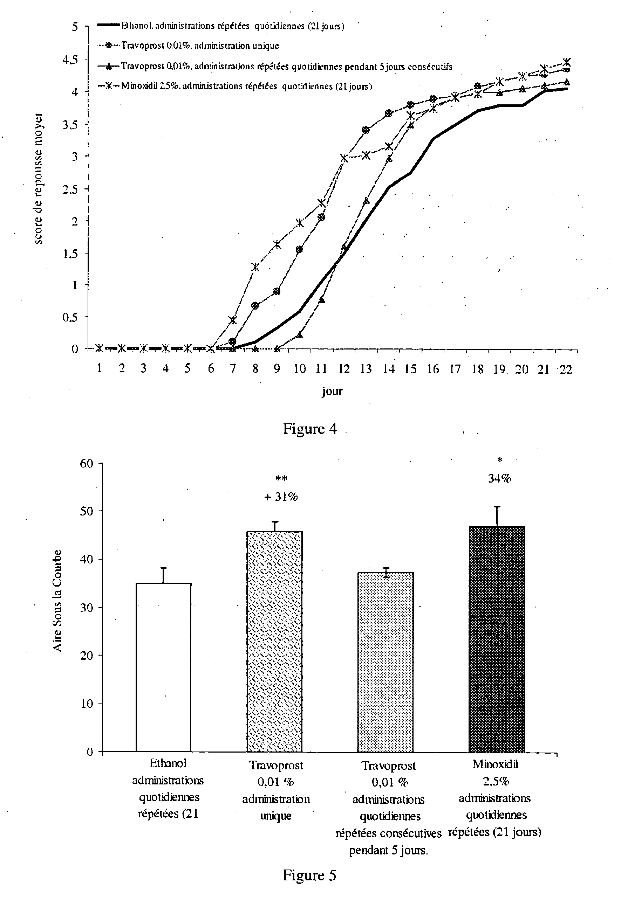

La figure 4 est un graphique comparant l'effet sur l'évolution de la repousse du poil chez la souris C57BL/6 du véhicule éthanol en administration répétée quotidienne pendant 21 jours, de Minoxidil à 2.5 % en administration répétée quotidienne pendant 21 jours, de Travoprost 0.01 % en administration répétée quotidienne pendant 5 jours consécutifs et de Travoprost 0.01 % en administration unique
La figure 5 est un graphe comparant l'effet sur l'évolution de la repousse du poil chez la souris C57BL/6 exprimé par l'aire sous la courbe du véhicule éthanol en administration répétée quotidienne pendant 21 jours, de Minoxidil à 2.5 % en administration répétée quotidienne pendant 21 jours, de Travoprost 0.01 % en administration répétée quotidienne pendant 5 jours consécutifs et de Travoprost 0.01 % en administration unique
   p>0.05=NS; 0.01<p<0.05=*; 0.001<p < 0.01 1 = **; p < 0.001 = ***.

### EXEMPLES:

### Exemple 1 : Follicules pileux humains en survie

### 1. METHODES :

Les follicules pileux humains sont disséqués en conservant le bulbe et les gaines conjonctives et épithéliales intacts afin d'être mis en culture dans un milieu approprié qui permet d'assurer la croissance du follicule *ex vivo* tout en conservant l'intégrité de sa structure en phase de croissance ou anagène (Stenn, Ann N Y Acad Sci. 1991 Dec 26;642:xi-xiii). La croissance des follicules pileux *in vitro* est alors évaluée à l'aide de la mesure de l'allongement de la gaine interne.

### A°/ Organoculture :

Les follicules pileux humains de type « terminal » sont isolés à partir d'un prélèvement de peau issu de chirurgie faciale (lifting). Pour cela, la peau est coupée en morceaux de 5*5mm environ. L'hypoderme est séparé du derme en utilisant une lame de scalpel. Les follicules pileux sont isolés de la partie hypodermique sous loupe binoculaire à l'aide de pinces fines. Seuls les follicules pileux en phase de croissance avec un bulbe et des gaines conjonctives et épithéliales intacts sont conservés pour l'étude.
Les follicules pileux ainsi isolés sont incubés à 37°C dans une atmosphère humide (5% CO2 et 95% air) et sont cultivés de façon individuelle en suspension pendant 7 jours dans du milieu de William's E supplémenté avec 2mM de L-Glutamine, 10µg/ml d'insuline, 10ng/ml d'hydrocortisone et 1% d'antibiotiques/antimitotiques. Le milieu de culture est renouvelé tous les 2 ou 3 jours.

### B°/ Traitement:

Le Travoprost sous sa forme acide est repris dans du DMSO à la concentration de 10⁻²M et conservé à -20°C jusqu'à utilisation.
Les follicules pileux en survie sont traités par le véhicule DMSO à la concentration finale de 0.1% ou par le Travoprost acide à la concentration de 1µM dans le DMSO (concentration finale de DMSO également à 0.1 %). Pour chaque condition, 22 follicules pileux sont traités individuellement suite à la mise en culture à J0. Dans le cas d'un traitement répété, le Travoprost acide à 1 µM dans le DMSO est renouvelé au moment du changement de milieu et ce pendant 7 jours. Dans le cas d'un traitement unique, le Travoprost acide à 1 µM dans le DMSO est ajouté dans le milieu de culture une seule fois à la mise en culture.

### C°/ Mesure de la croissance des follicules pileux in vitro :

La croissance des follicules pileux est déterminée par la mesure de l'élongation de la tige pilaire du jour J0 au jour J7. L'élongation de la tige pilaire est analysée en utilisant le logiciel d'analyse d'image Tina. Les mesures sont réalisées sur les follicules pileux isolés aux jours J0, J3, J5 et J7.

### D°/ Analyse statistique

Pour un follicule pileux:
- On considère la longueur moyenne post J0 (par défnition : la moyenne des longueurs mesurées aux jours suivant J0)
- La longueur moyenne post J0 est une fonction:
   - De la longueur initiale à J0
   - Du groupe de traitement
   - D'autres facteurs liés à la variabilité technologique ou biologique
- On veut estimer l'effet du traitement et lui associer une significativité - probabilité que le niveau estimé de l'effet puisse être le fruit du hasard, c'est-à-dire dans l'hypothèse (dite «nulle») où il n'y a pas d'effet réel.

A. Filtrage préliminaire : les follicules pileux présentant une morphologie « suspecte » à la fin de l'étude sont exclus de l'analyse. Dans cet exemple d'étude, 15 follicules (tous groupes confondus) pileux sur 66 mis en culture ont été retirés.
B. Modèle statistique : on suppose que la longueur moyenne post J0 est la somme de quatre composantes:
   - L'effet « naturel » du temps sur l'élongation de la tige pilaire en présence de DMSO.
   - L'effet complémentaire en présence du produit (qui peut-être positif ou négatif).
   - Un effet proportionnel à la longueur initiale (longueur à JO). Le coefficient de proportionnalité peut être dépendant du groupe.
   - Un effet aléatoire lié à la variabilité technologique ou biologique.
C. Test Anova robuste
   Certaines mesures dites « aberrantes » peuvent fortement perturber l'estimation des effets du modèle. On applique une méthode d'ANOVA robuste vis-à-vis de ces mesures: « LTS » (Least Trimmed Squares). Le principe de la méthode LTS est le suivant:
   1. On définit au départ une proportion fixe p0 d'observations que l'on souhaite ignorer dans l'estimation des effets (p0=10%) parmi toutes les observations présentes indépendamment du traitement (observations de l'étude entière). On note n0 le nombre d'observations correspondant.
   2. On sélectionne au hasard n0 points que l'on exclue du processus d'estimation.
   3. On estime les paramètres (effets) du modèle.
   4. Parmi les observations, exclues et non exclues, on identifie les n0 observations qui sont le moins bien prédites par le modèle.
   5. Les n0 observations les moins bien prédites trouvées dans l'étape 4, sont à leur tour exclues du processus d'estimation.
   6. On revient à l'étape 3. (estimation) jusqu'à ce que les observations exclues dans l'étape 5. demeurent inchangées (« convergence »).
   Il est à noter que la méthode d'exclusion est totalement indépendante de la question posée (effet significatif du traitement).
   Compte tenu de la sélection aléatoire effectuée dans l'étape 2 du processus LTS, la solution (en termes de points exclus après convergence) n'est généralement pas unique. En conséquence, l'estimation des effets du modèle dépend de la sélection aléatoire initiale.
   On répète un grand nombre de fois (200) le processus LTS en partant de sélections aléatoires différentes. Parmi les 200 modèles trouvés, on choisit celui qui minimise la différence entre les coefficients de proportionnalité associés à chacun des groupes qui relient la longueur initiale à la longueur moyenne post J0 (optimisation du parallélisme). Ce processus d'optimisation du parallélisme garantit que l'effet additif du traitement est identique quelle que soit la longueur initiale du follicule (pas d'interaction significative entre traitement et pente de la droite.)
   Ce processus a permis l'exclusion de 6 follicules sur un total de 51 follicules utilisés dans l'analyse statistique.
D. Tests inférentiels
   - Pour chacun des groupes traités, on teste la significativité de l'effet complémentaire du produit par rapport au groupe contrôle (construction des contrastes: Effet(Traité i) - Effet(Contrôle) pour chaque groupe traité i).
   - La significativité est ajustée par le nombre de tests effectués (=nombre de groupes traités).

### 2. RESULTATS :

Pour chaque traitement, l'élongation est exprimée en mm cumulés depuis le jour J0 [moyenne +/-SEM (n=22 à J0)]. L'aire sous la courbe (la somme des longueurs totales de chaque follicule pileux) est également calculée du jour J0 à J7 pour chaque condition [moyenne +/- SEM (n=22 à J0)].
Les résultats présentés sur les figures 1 et 2 et résumés dans le tableau ci-dessous montrent que comme décrit dans la littérature, l'élongation des follicules pileux après traitement par le véhicule DMSO est proche de 0.15 mm par jour. Un traitement unique par le Travoprost acide à la concentration de 1µM dans le DMSO induit une élongation de la tige pilaire des follicules pileux humains en survie supérieure à celle obtenue avec le véhicule DMSO. Cet effet apparait dès le troisième jour de traitement. La cinétique et l'amplitude de réponse observée après un traitement unique au moment de la mise en culture par le Travoprost acide à 1µM dans le DMSO est comparable à celle obtenue après un traitement répété durant les 7 jours de culture.

Les résultats de l'analyse statistique présentés sur la figure 3 montrent que l'effet inducteur du Travoprost après traitement unique ou répété est significativement différent de celui observé avec le véhicule DMSO.

| Traitement | Elongation à J7 (mm) | AUC (J0-J7) | Significativité |
|---|---|---|---|
| DMSO | 0,82 | 4,60 | |
| Travoprost à 1 µM en traitement répété | 1,23 | 5,82 | * |
| Travoprost à 1 µM en traitement unique | 1,27 | 5,83 | ** |

### 3. CONCLUSION:

Cet exemple démontre que le Travoprost en traitement répété induit de façon significative la croissance des follicules pileux humains *in vitro.* De façon surprenante, cet exemple montre également que le Travoprost en traitement unique induit la croissance des follicules pileux de façon similaire, en termes de cinétique et d'amplitude de réponse, que le Travoprost en traitement répété.
Ce résultat indique qu'un traitement répété chez l'Homme, c'est-à-dire quotidien, ne serait pas indispensable pour stimuler ou induire la croissance des follicules pileux.
L'effet du Travoprost a donc été évalué dans le modèle de repousse du poil chez la souris C57BL/6 afin de valider ces résultats *in vivo.*

### Exemple 2 : Modèle de repousse du poil chez la souris C57BL/6 :

### 1. METHODES :

Les souris C57BL/6 représentent un modèle particulièrement utile pour l'étude de la croissance pilaire :
Chez les souris C57BL/6 le pelage est noir. Au niveau du tronc des animaux, les mélanocytes sont présents exclusivement dans les follicules pileux et la production de pigment est strictement corrélée à la phase anagène du cycle pilaire (Slominski A et Paus R., J. Invest. Dermatol. 10 : 90S-97S, 1993).
Chez les souris jeunes, les cycles pilaires ne sont pas en phase sur tous le corps mais sont synchronisés au niveau d'une région selon une vague rostro-caudale (Hale P. A. and Ebling F.J., J. Exp. Zool., 191 : 49-62). A 42 jours, les follicules pileux de la région dorsale entrent en phase télogène. (Chase HB. Physiol Rev 34: 113-126, 195).
Ainsi, après tonte et rasage du dos des souris C57BL/6 âgées de 42 jours, la repousse peut être aisément visualisée avant que les tiges pilaires n'apparaissent à la surface par l'aspect gris que la peau prend. Le délai d'apparition de la couleur grise de la peau et le délai d'apparition des tiges pilaires après tonte et rasage représentent des critères rendant compte de la vitesse d'entrée du follicule pileux en phase anagène et de la vitesse de croissance de la tige pilaire. Ces deux critères seront donc utilisés pour évaluer l'activité des composés testés sur la croissance pilaire.

### A°/ Modèle:

Les souris, C57BL/6, mâles sont âgées de âgés 42 jours au début de l'étude. Les groupes sont formés de 10 animaux.
Au jour J0, le dos des animaux est tondu avec une tondeuse « minicut » munie d'un sabot (préservation de 4 mm de tige pilaire) et rasé avec un rasoir Braun. Vingt-quatre heures après la tonte et le rasage, au jour J1 de l'étude, une observation clinique est effectuée et ne sont sélectionnées pour la mise en étude que les souris présentant une peau uniformément rose, et indemne de lésions cutanées.
L'étude s'effectue sur 21 jours.

| Jour de l'étude | Actions |
|---|---|
| J-5 | Réception des animaux et mise en acclimatation |
| J0 | Anesthésie, tonte et rasage des animaux |
| J1 | Sélection, randomisation et mise en étude des animaux |
| J1 à J21 | Traitement au niveau de la zone tondue et rasée |
| J1 à J22 | Observations cliniques |
| J1 à J21 | Evaluation de la croissance pilaire |
| J22 | euthanasie des animaux |

La croissance pilaire est évaluée selon les critères définis dans :

| Scores | Aspect clinique |
|---|---|
| 0 | Peau uniformément rose |
| 1 | Apparition de taches de couleur grise sur la surface tondue et rasée |
| 2 | Apparition des premières tiges pilaires sur la surface tondue et épilée |
| 3 | Couleur grise sur l'ensemble de la surface tondue et épilée |
| 4 | Présence de tiges pilaires sur l'ensemble de la surface tondue et épilée |
| 5 | Repousse uniforme des poils entre la surface tondue / épilée et la surface non épilée |

### B°/ Traitement:

Le Minoxidil en solution dans l'éthanol à 2.5 % est utilisé comme contrôle positif de la repousse du poil.
Le Travoprost sous sa forme isopropyl ester est utilisé à 0.01 % en solution dans l'éthanol.
Le Travoprost, le Minoxidil et le véhicule sont administrés par voie topique à l'aide d'une pipette, à raison de 50µl par application.
Le Minoxidil et le véhicule sont administrés de façon quotidienne répétée du jour 1 au jour 21 de l'étude. Deux traitements différents sont effectués avec le Travoprost : un premier groupe recevant une administration unique au jour 1 de l'étude, un second groupe recevant cinq administrations répétées quotidiennes et consécutives du jour 1 au jour 5 de l'étude.

### C°/ Evaluation de la repousse et Analyse statistique:

Les calculs suivants sont effectués :
- Moyenne ± esm des scores par jour pour chaque groupe traité.
- Aires sous la courbe (AUC) des scores de repousse par animal de J1 à J22.
- Moyenne ± esm des AUC par groupe de traitement.
- Pourcentage d'augmentation des AUC pour chaque groupe traité versus le groupe véhicule.
L'AUC est obtenue en effectuant la somme des aires des rectangles entre le premier et le dernier jour de l'étude. Les différents groupes de traitements sont comparés au groupe véhicule sur ce paramètre au moyen d'un test t de Student.

### 2. RESULTATS :

Les résultats présentés dans les figures 4 et 5 et dans le tableau de résultats ci-dessous montrent que le Minoxidil en administration quotidienne répétée pendant 21 à la dose de 2.5 % accélère la repousse du poil par rapport au véhicule éthanol. De façon surprenante, une administration unique de Travoprost à 0.01 %, accélère significativement la repousse du poil par rapport au véhicule avec une amplitude de réponse (31 %) identique à celle obtenu après traitement quotidien répétée par Minoxidil à 2.5 % (30 %).
Contre toute attente, des administrations répétées quotidiennes et consécutives pendant 5 jours de Travoprost à 0,01 % n'accélèrent pas la repousse des poils.

| Traitement | Repousse du poil versus éthanol ΔAUC (J1-J22) | p value (test T de Student) | Significativité |
|---|---|---|---|
| Minoxidil à 2.5 % administrations quotidiennes répétées (21 jours) | +34 % | 0.035 | * |
| Travoprost à 0.01 % administrations répétées quotidiennes (5 jours) | +7 % | 0.506 | NS |
| Travoprost à 0.01 % administration unique | +31 % | 0.011 | ** |

### 3. CONCLUSION:

Ce deuxième exemple démontre, de façon surprenante, que le Travoprost après administration unique accélère significativement la repousse du poil chez la souris *in vivo*. Cet effet est comparable à celui observé avec le composé de référence Minoxidil utilisé en administration quotidienne répétée. Toutefois, contre toute attente, le Travoprost utilisé en administrations répétées quotidiennes consécutives pendant 5 jours, est moins efficace sur la repousse du poil *in vivo*. Une administration unique de Travoprost *in vivo* est donc plus efficace sur l'induction de la croissance des follicules pileux qu'une administration répétée quotidiennement.
Les exemples 1 et 2 montrent donc que le Travoprost induit la croissance du follicule pileux *in vitro* et *in vivo* de façon comparable voire plus efficace après administration unique que quotidienne répétée.

L'ensemble de ces travaux permet de soutenir l'utilisation de Travoprost chez l'Homme suivant une posologie adaptée, à savoir une application non quotidienne selon la présente invention, qui permet une efficacité maximale du composé pour obtenir une stimulation ou induction de la croissance des follicules pileux.

## Revendications

1. Utilisation du Travoprost pour la préparation d'un médicament pour stimuler ou induire la croissance et/ou diminuer la chute et/ou augmenter la densité et/ou réduire l'hétérogénéité du diamètre des cheveux et/ou des poils chez l'être humain, **caractérisé en ce que** le régime d'administration du médicament comprend un intervalle de temps entre deux applications supérieur à 24 heures, et est choisi parmi une application tous les deux, trois, quatre, cinq, six ou sept jours, une application hebdomadaire, une application bi- ou tri-hebdomadaire, une application mensuelle, ou une application bi ou tri-mensuelle, et **en ce que** le médicament comprend une quantité de Travoprost comprise entre 0,005 et 0,01%.

2. Utilisation selon la revendication 1, dans laquelle le médicament est destiné a être applique de manière topique.

3. Utilisation selon l'une quelconque des revendications 1 à 2, dans laquelle le médicament est sous la forme d'une lotion, d'une crème, d'une émulsion, d'une pommade, d'un mascara, d'une poudre ou d'un shampoing ou après-shampoing.

4. Travoprost pour utilisation dans une méthode de traitement de l'alopécie androgénique, **caractérisée en ce qu'**une composition de soin capillaire comprenant du Travoprost, est appliquée à un homme nécessitant un tel traitement, ladite composition étant destinée à être appliquée avec un intervalle de temps entre deux applications supérieur à 24 heures, et choisi parmi une application tous les deux, trois, quatre, cinq, six ou sept jours , une application hebdomadaire, bi- hebdomadaire, tri-hebdomadaire, mensuelle, bi- ou tri- mensuelle, et comprend une quantité de Travoprost comprise entre 0,005 et 0,01%.

## Patentansprüche

1. Verwendung von Travoprost für die Herstellung eines Arzneimittels zum Stimulieren oder Induzieren des Wachstums und/oder Verringern des Ausfallens und/oder Erhöhen der Dichte und/oder Reduzieren der Unterschiedlichkeit des Durchmessers von Haar bzw. Kopfhaar beim Menschen, **dadurch gekennzeichnet, dass** das Verabreichungsschema des Arzneimittels ein Zeitintervall zwischen zwei Applikationen von mehr als 24 Stunden umfasst und aus einer Applikation alle zwei, drei, vier, fünf, sechs oder sieben Tage, einer wöchentlichen Applikation, einer Applikation alle zwei oder drei Wochen, einer monatlichen Applikation oder einer Applikation alle zwei oder drei Monate ausgewählt ist und dass das Arzneimittel eine Travoprost-Menge zwischen 0,005 und 0,01% umfasst.

2. Verwendung nach Anspruch 1, wobei das Arzneimittel zur topischen Applikation bestimmt ist.

3. Verwendung nach einem der Ansprüche 1 bis 2, wobei das Arzneimittel in Form einer Lotion, einer Creme, einer Emulsion, einer Salbe, einer Wimperntusche, eines Puders, eines Schampoos oder eines Pflegemittels nach dem Schamponieren vorliegt.

4. Travoprost zur Verwendung in einem Verfahren zur Behandlung von androgener Alopecia, **dadurch gekennzeichnet, dass** eine Travoprost enthaltende Haarpflegezusammensetzung einem Mann, der solch einer Behandlung bedarf, appliziert wird, wobei die Zusammensetzung mit einem Zeitintervall zwischen zwei Applikationen von mehr als 24 Stunden zu applizieren ist und aus einer Applikation alle zwei, drei, vier, fünf, sechs oder sieben Tage, einer wöchentlichen, zweiwöchentlichen oder dreiwöchentlichen Applikation, einer monatlichen, zweimonatlichen oder dreimonatlichen Applikation ausgewählt ist und eine Travoprost-Menge zwischen 0,005 und 0,01% umfasst.

## Claims

1. Use of travoprost in the preparation of a medicament for stimulating or inducing the growth and/or decreasing the loss and/or increasing the density and/or reducing the heterogeneity in the diameter of the head hairs and/or non-head hairs in human beings, **characterized in that** the regime for administration of the medicament comprises a time interval between two applications of greater than 24 hours, and is chosen from an application every two, three, four, five, six or seven days, a weekly application, a twice or thrice weekly application, a monthly application or a twice or thrice monthly application, and **in that** the medicament comprises an amount of travoprost of between 0.005 and 0.01%.

2. Use according to Claim 1, in which the medicament is intended to be applied topically.

3. Use according to either one of Claims 1 and 2, in which the medicament is in the form of a lotion, a cream, an emulsion, a salve, a mascara, a powder or a shampoo or conditioner.

4. Travoprost for use in a method for the treatment of androgenic alopecia, **characterized in that** a hair care composition comprising travoprost is applied to a man requiring such a treatment, the said composition being intended to be applied with a time interval between two applications of greater than 24 hours, and chosen from an application every two, three, four, five, six or seven days, a weekly, twice weekly, thrice weekly, monthly, twice monthly or thrice monthly application and comprises an amount of travoprost of between 0.005 and 0.01%.
